# EUROPÄISCHE PATENTSCHRIFT

(11) **EP 1 796 766 B1**
(45) Veröffentlichungstag und Bekanntmachung des Hinweises auf die Patenterteilung: **26.10.2011**
(21) Anmeldenummer: 05755996.5
(22) Anmeldetag: 03.06.2005
(51) Int. Cl.: A61M 16/00

(54) **VORRICHTUNG ZUR BEATMUNG**
RESPIRATORY DEVICE
DISPOSITIF RESPIRATOIRE

(30) Priorität: 20.08.2004 DE 102004040659
(43) Veröffentlichungstag der Anmeldung: 20.06.2007
(73) Patentinhaber: Weinmann Geräte für Medizin GmbH & Co. KG, 22525 Hamburg (DE)
(72) Erfinder: SCHWAIBOLD, Matthias, 76137 Karlsruhe (DE); SCHÖLLER, Bernd, 76135 Karlsruhe (DE)
(74) Vertreter: Hansmann, Dierk
(86) Internationale Anmeldenummer: PCT/DE2005/000996
(87) Internationale Veröffentlichungsnummer: WO 2006/021169

(56) Entgegenhaltungen:
- EP-A- 0 722 747
- EP-A2- 1 136 094
- EP-A2- 1 136 094
- WO-A-02/28281
- WO-A-2004/084980
- US-A- 5 953 713
- US-A1- 2004 074 492

## Beschreibung

Die Erfindung betrifft eine Vorrichtung zur Beatmung, die eine mit einer Beatmungsmaske verbindbare Druckgasquelle, eine Steuerung für die Druckgasquelle sowie eine Meßeinrichtung zur Erfassung mindestens eines Atemparameters aufweist und bei der die Steuerung eine Adaptionseinrichtung zur Veränderung des von der Druckgasquelle bereitgestellten Druckes in Abhängigkeit von einer Auswertung des gemessenen Atemparameters aufweist sowie bei der die Steuerung einen Analysator zur Erfassung mindestens eines Artefaktes aufweist und der Analysator derart mit der Steuerung gekoppelt ist, daß bei der Erkennung des Artefaktes eine von der Adaptionseinrichtung vorgegebene Druckänderung vermieden ist, sowie bei der der Analysator zur Auswertung eines Flowverlaufes ausgebildet ist und bei der der Analysator zur Auswertung eines Druckverlaufes ausgebildet ist, sowie bei der die Steuerung zur Durchführung einer APAP-Beatmung ausgebildet ist und ein Verfahrensablauf bei der Erkennung und steuerungstechnischen Berücksichtigung von Artefakten derart erfolgt, daß zunächst eine Erkennung von Inspirations- und Expirationsphasen durch Auswertung eines mit dem Flow im zusammenhang stehenden Signals oder durch Auswertung eines mit Druckschwankungen im Zusammenhang stehenden Signals durchgeführt wird und bei dem nach der Durchführung einer Messung ein Vergleich einer gemessenen Amplitude oder einer gemessenen Leistung mit einem aus vorangegangenen Expirationsphasen oder Inspirationsphasen abgeleiteten Referenzwert erfolgt, wobei bei einer Feststellung einer signifikanten Änderung ein oder mehrere Artefakttypen erkannt werden.

Derartige Vorrichtungen werden typischerweise für die Steuerung von sogenannten APAP-Geräten verwendet. Diese Geräte sind mit Druckgasquellen ausgestattet, die über einen Beatmungsschlauch und eine Beatmungsmaske mit den Atemwegen eines Patienten verbunden sind. Eine Steuerung der Druckgasquelle erfolgt in der Regel derart, daß in Abhängigkeit von einem gemessenen Atemparameter, beispielsweise dem Flow oder dem Druck, eine spezifische Drucksteuerung vorgenommen wird.

Es zeigt sich, daß zusätzlich zu den typischen Veränderungen des Atemparameters, der die steuerungstechnisch festgelegten Druckerhöhungen bzw. Druckabsenkungen auslöst, zusätzliche Veränderungen des Atemparameters in Abhängigkeit von Störungen auftreten. Diese störungsbedingten Druckerhöhungen bzw. Druckabsenkungen führen dazu, daß von einer optimalen Gerätesteuerung abgewichen wird.

In der EP-A-0722747 wird bereits eine Vorrichtung zur Beatmung beschrieben, die eine Druckgasquelle, eine Steuerung, eine Adaptionseinrichtung und eine Meßeinrichtung aufweist. Die Steuereinrichtung ist dafür geeignet, unterschiedliche Artefakte zu erfassen. Nach der Erkennung eines Artefaktes erfolgt eine Veränderung der zugeordneten Gerätefunktion.

Aus der EP 11 36 094 ist bereits ein Verfahren zur Steuerung eines Beatmungsgerätes sowie eine entsprechende Vorrichtung zur Überwachung bekannt. Der Atemgasströmung wird hierbei ein spezieller Druckverlauf als Testsignal überlagert und ein Antwortsignal auf dieses Testsignal wird ausgewertet. Aufgrund der Signalauswertung ist es unter anderem auch möglich, Rückschlüsse auf einen Gerätezustand zu gewinnen und gegebenenfalls Funktionsstörungen zu erkennen.

Aufgabe der vorliegenden Erfindung ist es, eine Vorrichtung der einleitend genannten Art derart zu konstruieren, daß auch beim Auftreten von Störungen eine optimale Gerätesteuerung gewährleistet ist.

Diese Aufgabe wird erfindungsgemäß dadurch gelöst, daß eine Bandpassfilterung für das gemessene Drucksignal verwendet ist, von der ein Frequenzband derart definiert ist, daß eine Amplitude einer vom Gerät erzeugten Volumenschwingung gemessen wird, wobei die Volumenschwingung durch eine Gebläseansteuerung erzeugt ist und daß eine Auswertung bei der Störungserkennung derart durchführbar ist , daß einzelne oder kumulierte exspiratorische Engungen als Artefakte bewertet werden.

Bei der erfindungsgemäßen Vorrichtung wird ausgenutzt, daß spezifische Artefakte zu typischen Beeinflussungen des meßtechnisch erfaßten Atemparameters führen. Die entsprechenden typischen zeitlichen Verläufe des Atemparameters, die einem bestimmten Artefakt zugeordnet sind, ermöglichen eine automatische Auswertung des Signalverlaufes hinsichtlich der jeweiligen Verläufe und somit eine steuerungstechnische Identifikation des jeweiligen Artefaktes.

Entsprechende spezifische und am Signalverlauf erkennbare Artefakte sind beispielsweise Mundexpiration, Mundatmung, Leckage, Schlucken, Sprechen oder Husten. Bei einer automatischen Erkennung derartiger Ereignisse ist es möglich, die Drucksteuerung derart zu modifizieren, daß diejenigen meßtechnischen Parameter, die in einem Normalzustand von der Steuerung für eine Druckerhöhung oder eine Druckabsenkung ausgewertet werden und die bei einem Auftreten des jeweiligen Artefaktes nicht mehr zuverlässig ausgewertet werden können, für die Zeitdauer des Auftretens des Artefaktes von der Steuerung nicht berücksichtigt werden.

Ein typischer Verfahrensablauf erfolgt dadurch, daß die Steuerung zur Durchführung einer APAP-Beatmung ausgebildet ist.

Gemäß einer Ausführungsform ist vorgesehen, daß der Analysator zur Auswertung eines Flowverlaufes ausgebildet ist.

Darüber hinaus ist auch daran gedacht, daß der Analysator zur Auswertung eines Druckverlaufes ausgebildet ist.

Eine Verfahrensvariante besteht darin, daß der Analysator zur Auswertung von Inspirationsphasen ausgebildet ist.

Darüber hinaus ist es auch möglich, daß der Analysator zur Auswertung von Exspirationsphasen ausgebildet ist.

Ein einfaches Auswertungsprinzip besteht darin, daß der Analysator zur Auswertung von Amplitudenwerten ausgebildet ist.

Darüber hinaus ist es auch möglich, daß der Analysator zur Auswertung von Leistungswerten ausgebildet ist.

Gemäß einer weiteren Ausführungsvariante ist vorgesehen, daß der Analysator einen Referenzwertkomperator aufweist.

Eine frequenzabhängige Signalauswertung wird dadurch unterstützt, daß eine Bandpaßfilterung des gemessenen Drucksignals durchgeführt wird.

Insbesondere ist daran gedacht, daß ein Frequenzband bei der Bandpaßfilterung derart definiert wird, daß eine Amplitude einer vom Gerät erzeugten Volumenschwingung gemessen wird.

Ein an die Bandpaßfilterung angepaßtes Anregungssignal kann dadurch bereitgestellt werden, daß die Volumenschwingung durch eine Membranpumpe erzeugt wird.

Gemäß einer anderen Ausführungsform ist es möglich, daß die Volumenschwingung durch eine Gebläseansteuerung erzeugt wird.

Ein guter Kompromiß zwischen einer einfachen gerätetechnischen Realisierbarkeit und einer guten Auswertbarkeit des Anregungssignals besteht darin, daß eine Volumenschwingung mit einer Frequenz von etwa 20 Hz erzeugt wird.

Gemäß einem typischen Auswertungsverfahren ist vorgesehen, daß eine Auswertung derart durchgeführt wird, daß durch einen expiratorischen Anstieg der Druckschwingungsamplitude im Vergleich zu einem Referenzwert eine Erkennung einer expiratorischen Verengung der Atemwege durchgeführt wird.

Eine besondere Variante der Störungserkennung besteht darin, daß eine Auswertung derart durchgeführt wird, daß einzelne oder kumulierte exspiratorische Verengungen als Artefakte bewertet werden.

Ein vorteilhaftes Signalverhalten auch bei einem Auftreten von Störungen wird dadurch unterstützt, daß bei einer steuerungstechnischen Erkennung eines Artefaktes eine Ereigniserkennung und eine Druckreaktion deaktiviert wird.

Bei einer Auswertung der Häufigkeit des Auftretens von Artefakten ist es möglich, die Qualität des verwendeten Maskentyps zu beurteilen. Dies ermöglicht eine Qualitätskontrolle sowohl bei APAP- als auch bei CPAP- und Bilevel-Anwendungen.

In den zeichnungen sind Ausführungsbeispiele der Erfindung schematisch dargestellt. Es zeigen:
- Fig. 1: eine perspektivische Darstellung eines Beatmungsgerätes mit Verbindungsschlauch zu einer Beatmungsmaske und
- Fig. 2: Signalverläufe bei der Auswertung einer Druckschwankung, die an der Beatmungsmaske gemessen wird, mit Erkennung von Mundexpirationsphasen.

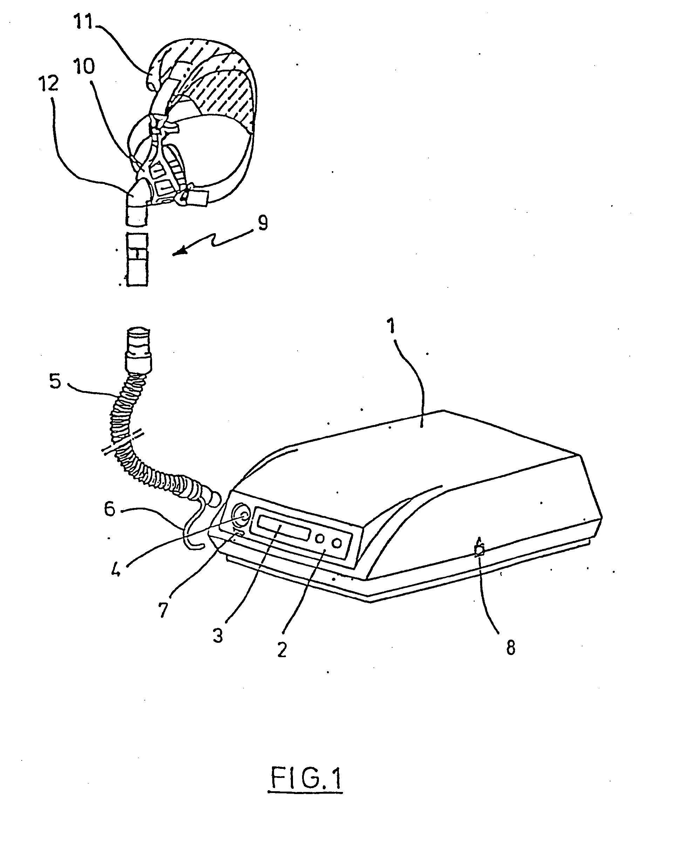

Fig. 1 zeigt den grundsätzlichen Aufbau einer Vorrichtung zur Beatmung. Im Bereich eines Gerätegehäuses (1) mit Bedienfeld (2) sowie Anzeige (3) ist in einem Geräteinnenraum eine Atemgaspumpe angeordnet. Über eine Kopplung (4) wird ein Verbindungsschlauch (5) angeschlossen. Entlang des Verbindungsschlauches (5) kann ein zusätzlicher Druckmeßschlauch (6) verlaufen, der über einen Druckeingangsstutzen (7) mit dem Gerätegehäuse (1) verbindbar ist. Zur Ermöglichung einer Datenübertragung weist das Gerätegehäuse (1) eine Schnittstelle (8) auf.

Im Bereich einer dem Gerätegehäuse (1) abgewandten Ausdehnung des Verbindungsschlauches (5) ist ein Ausatmungselement (9) angeordnet. Ebenfalls kann ein Ausatemventil verwendet werden.

Fig. 1 zeigt darüber hinaus eine Beatmungsmaske (10), die als Nasalmaske ausgebildet ist. Eine Fixierung im Bereich eines Kopfes eines Patienten kann über eine Kopfhaube (11) erfolgen. Im Bereich ihrer dem Verbindungsschlauch (5) zugewandten Ausdehnung weist die Beatmungsmaske (10) ein Kupplungselement (12) auf.

Ein prinzipieller Verfahrensablauf bei der Erkennung und steuerungstechnischen Berücksichtigung von Artefakten kann derart erfolgen, daß zunächst eine Erkennung von Inspirations- und Expirationsphasen durch Auswertung eines mit dem Flow im Zusammenhang stehenden Signals oder durch Auswertung eines mit Druckschwankungen im Zusammenhang stehenden Signals durchgeführt wird. Es erfolgt eine Messung von Amplitude oder Leistung von expiratorischen Druckschwankungen, die entweder vom Gerät erzeugt werden oder im Bereich des Patienten entstehen. Die vom Gerät erzeugten Druckschwankungen können durch ein moduliertes Drucksignal gebildet werden, beispielsweise einem ODS-oder FOT-Signal. Vom Patienten erzeugte Druckschwankungen können beispielsweise Zischgeräusche bei der Mundexpiration oder Leckagen sein.

Nach der Durchführung der Messung erfolgt ein Vergleich der gemessenen Amplitude oder der gemessenen Leistung mit einem aus vorangegangenen Expirationsphasen oder Inspirationsphasen abgeleiteten Referenzwert. Bei einer Feststellung einer signifikanten Änderung erfolgt die Erkennung eines oder mehrerer Artefakttypen.

Optional ist es möglich, eine Unterscheidung der Artefakte anhand der Höhe der Änderung, anhand der Anzahl der konsekutiven Atemzüge mit Änderung oder durch eine Auswertung dahingehend vorzunehmen, ob die jeweilige Änderung nur in expiratorischen oder auch in inspiratorischen Phasen im Vergleich zum vorangegangenen Atemzug auftritt.

Ebenfalls ist es optional möglich, eine zusätzliche Erhöhung der Analysequalität durch eine Auswertung des Flowverlaufs vorzunehmen, wenn beispielsweise die expiratorische Halbwelle bei der Mundexpiration fehlt.

Es erfolgt anschließend eine Änderung der Druckreaktion des Gerätes für eine veränderte Reaktion auf die gegebenen Ereignisse, beispielsweise eine Aussetzung der Erkennung bestimmter Ereignisse. Ebenfalls ist es möglich, eine Änderung von Wartezeiten vor einer Druckabsenkung oder einer Druckanhebung vorzunehmen. Ebenfalls können die Anhebungs- und Absenkgeschwindigkeiten variiert werden.

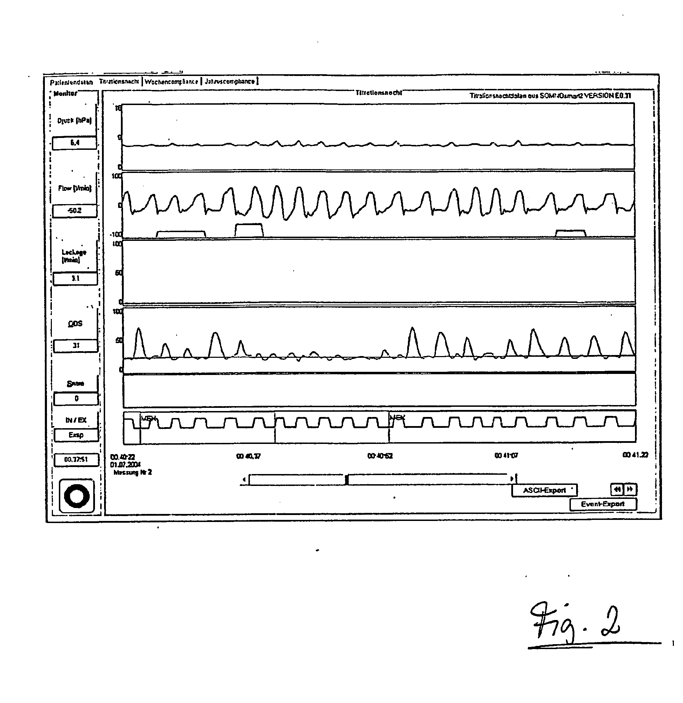

Fig. 2 zeigt ein Ausführungsbeispiel, bei der eine Druckschwingung an der Beatmungsmaske gemessen und im Gerät erzeugt wird. Die Druckschwingung kann als ODS-oder FOT-Signal vorliegen.

Es erfolgt ein vergleich der exspiratorisch gemessenen Amplitude der Druckschwingung mit der inspiratorisch gemessenen Amplitude der Druckschwingung. Hierbei wird ein vergleich der Mittelwerte der beiden Atemphasen durchgeführt.

Falls der expiratorische Wert um mindestens einen festgelegten ersten Betrag höher ist als der inspiratorische Wert, so erfolgt eine Erkennung eines einzelnen expiratorischen Artefaktes, beispielsweise Schlucken, Husten oder Sprechen.

Falls die expiratorischen Werte um mindestens einen festgelegten zweiten Betrag höher als die inspiratorischen Werte für eine vorher festgelegte Anzahl von Atemzügen sind, erfolgt eine Erkennung von Mundatmung oder Mundexpiration.

In Abhängigkeit vom Auswertungsergebnisse erfolgt eine Unterdrückung der Auswertung des inspiratorischen Flowvolumens und somit eine Unterdrückung der Hypobnoeerkennung und ebenfalls eine Unterdrückung der Auswertung der inspiratorischen Amplitude der Druckschwingung im Zusammenhang mit der Oszilloresistometrie, da unter Mundatmung bzw. bei Auftreten eines expiratorischen Artefaktes eine entsprechende Reaktion unzuverlässig ist.

Gemäß dem Verlauf in Fig. 2 erfolgt eine Erkennung von Mundexpirationsphasen im IN/EX-Kanal. Die betreffende Mund-exspirationsphasen sind in Fig. 2 durch die gestrichelten Linien markiert.

Gemäß einem anderen Ausführungsbeispiels erfolgt eine Messung der natürlichen Druckschwingungen in einem bestimmten Frequenzband durch Bandpaßfilterung des gemessenen Drucksignals, um Schnarch- oder Zischgeräusche zu erkennen. Bei einem expiratorischen Anstieg erfolgt eine Erkennung von Mundexpirationen, bei einem inspiratorischen oder expiratorischen Anstieg die Erkennung einer Leckage. Es erfolgt auch in jedem Fall wieder eine Unterdrückung von Ereignissen bzw. eine Verschiebung von Schwellwerten, beispielsweise zur Schnarcherkennung.

## Patentansprüche

1. Vorrichtung zur Beatmung, die eine mit einer Beatmungsmaske verbindbare Druckgasquelle, eine Steuerung für die Druckgasquelle sowie eine Meßeinrichtung zur Erfassung mindestens eines Atemparameters aufweist und bei der die Steuerung eine Adaptionseinrichtung zur Veränderung des von der Druckgasquelle bereitgestellten Druckes in Abhängigkeit von einer Auswertung des gemessenen Atemparameters aufweist sowie bei der die Steuerung einen Analysator zur Erfassung mindestens eines Artefaktes aufweist und der Analysator derart mit der Steuerung gekoppelt ist, daß bei der Erkennung des Artefaktes eine von der Adaptionseinrichtung vorgegebene Druckänderung vermieden ist, sowie bei der der Analysator zur Auswertung eines Flowverlaufes ausgebildet ist und bei der der Analysator zur Auswertung eines Druckverlaufes ausgebildet ist, sowie bei der die Steuerung zur Durchführung einer APAP-Beatmung ausgebildet ist und ein Verfahrensablauf bei der Erkennung und steuerungstechnischen Berücksichtigung von Artefakten derart erfolgt, daß zunächst eine Erkennung von Inspirations- und Expirationsphasen durch Auswertung eines mit dem Flow im Zusammenhang stehenden Signals oder durch Auswertung eines mit Druckschwankungen im Zusammenhang stehenden Signals durchgeführt wird und bei dem nach der Durchführung einer Messung ein Vergleich einer gemessenen Amplitude oder einer gemessenen Leistung mit einem aus vorangegangenen Expirationsphasen oder Inspirationsphasen abgeleiteten Referenzwert erfolgt, wobei bei einer Feststellung einer signifikanten Änderung ein oder mehrere Artefakttypen erkannt werden, **dadurch gekennzeichnet, daß** eine Bandpassfilterung für das gemessene Drucksignal verwendet ist, von der ein Frequenzband derart definiert ist, daß eine Amplitude einer vom Gerät erzeugten Volumenschwingung gemessen wird, wobei die Volumenschwingung durch eine Gebläseansteuerung erzeugt ist und daß eine Auswertung bei der Störungserkennung derart durchführbar ist, daß einzelne oder kumulierte exspiratorische Engungen als Artefakte bewertet werden.

2. Vorrichtung nach Anspruche 1, **dadurch gekennzeichnet, daß** der Analysator zur Auswertung von Inspirationsphasen ausgebildet ist.

3. Vorrichtung nach einem der Ansprüche 1 oder 2, **dadurch gekennzeichnet, daß** der Analysator zur Auswertung von Exspirationsphasen ausgebildet ist.

4. Vorrichtung nach einem der Ansprüche 1 bis 3, **dadurch gekennzeichnet, daß** der Analysator zur Auswertung von Amplitudenwerten ausgebildet ist.

5. Vorrichtung nach einem der Ansprüche 1 bis 3, **dadurch gekennzeichnet, daß** der Analysator zur Auswertung von Leistungswerten ausgebildet ist.

6. Vorrichtung nach einem der Ansprüche 1 bis 5, **dadurch gekennzeichnet, daß** der Analysator einen Referenzwertkomperator aufweist.

## Claims

1. A device for artificial respiration, which has a compressed gas source which may be connected to a respiratory mask, a control means for the compressed gas source and a measuring device for recording at least one breathing parameter, and in which the control means has an adaptation device for altering the pressure provided by the compressed gas source depending on an evaluation of the measured breathing parameter and in which the control means has an analyser for recording at least one artefact and the analyser is coupled to the control means in such a way that, upon recognition of the artefact, a change in pressure specified by the adaptation device is prevented, and in which the analyser is designed to evaluate a flow profile and in which the analyser is designed to evaluate a pressure profile, and in which the control means is designed to carry out APAP ventilation and a process sequence takes place upon the recognition and controlled observance of artefacts in such a way that recognition of inspiration and expiration phases is firstly carried out by evaluating a signal associated with the flow or by evaluating a signal associated with pressure fluctuations and in which, after taking a measurement, a measured amplitude or a measured output is compared with a reference value derived from previous expiration phases or inspiration phases wherein, upon establishing a significant change, one or more artefact types are recognised, **characterised in that** band-pass filtering is used for the measured pressure signal, from which a frequency band is defined in such a way that an amplitude of a volume oscillation generated by the device is measured, wherein the volume oscillation is generated by a blower control and **in that** an evaluation upon recognition of a malfunction can be carried out in such a way that individual or cumulative expiratory narrowings are assessed as artefacts.

2. A device according to Claim 1, **characterised in that** the analyser is designed to evaluate inspiration phases.

3. A device according to one of Claims 1 and 2, **characterised in that** the analyser is designed to evaluate expiration phases.

4. A device according to one of Claims 1 to 3, **characterised in that** the analyser is designed to evaluate amplitude values.

5. A device according to one of Claims 1 to 3, **characterised in that** the analyser is designed to evaluate output values.

6. A device according to one of Claims 1 to 5, **characterised in that** the analyser has a reference value comparator.

## Revendications

1. Dispositif respiratoire, qui est doté d'une source de gaz sous pression pouvant être reliée à un masque respiratoire, d'une commande de la source de gaz comprimé, ainsi que d'un dispositif de mesure pour la saisie d'au moins un paramètre de respiration, et dans lequel la commande est dotée d'un système d'adaptation pour la modification de la pression, qui est mise à disposition par la source de gaz comprimé en fonction d'une évaluation du paramètre de respiration mesuré, et dans lequel la commande est dotée d'un analyseur pour la saisie d'au moins un artefact, et l'analyseur est couplé avec la commande de sorte que, lors de la détection d'un artefact, une modification de la pression, prédéterminée par le système d'adaptation, est évitée, ainsi que dans lequel l'analyseur est conçu pour l'évaluation d'une évolution de la pression, ainsi que dans lequel la commande est conçue pour la réalisation d'une ventilation APAP, et un déroulement du procédé est effectué, lors de la détection et de la prise en considération des artefacts au point de vue technique de commande, de sorte qu'une détection de phases d'inspiration et d'expiration est d'abord effectuée par évaluation d'un signal, qui se rapporte au débit, ou par évaluation d'un signal, qui se rapporte aux fluctuations de pression, et dans lequel, après l'exécution d'une opération de mesure, une comparaison est effectuée entre une amplitude mesurée ou un débit mesuré et une valeur de référence, qui est dérivée de phases d'expiration ou de phases d'inspiration précédentes, sachant que, lors de la constatation d'une modification significative, un ou plusieurs types d'artefacts est / sont détectés, **caractérisé en ce que**, pour le signal de pression mesuré, on utilise une filtration passe bande, par laquelle une bande de fréquences est définie de sorte qu'une amplitude d'une oscillation de volume, générée par un appareil, est mesurée, sachant que l'oscillation de volume est générée par excitation d'une soufflante, et qu'une évaluation peut être effectuée, lors de la détection de perturbations, de sorte que des étranglements expiratoires, individuels ou cumulés, soient évalués en tant qu'artefacts.

2. Masque respiratoire selon la revendication 1, **caractérisé en ce que** l'analyseur est conçu pour l'évaluation de phases d'inspiration.

3. Dispositif respiratoire selon l'une des revendications 1 ou 2, **caractérisé en ce que** l'analyseur est conçu pour l'évaluation de phases d'expiration.

4. Dispositif respiratoire selon l'une des revendications 1 à 3, **caractérisé en ce que** l'analyseur est conçu pour l'évaluation de valeurs d'amplitude.

5. Dispositif respiratoire selon l'une des revendications 1 à 3, **caractérisé en ce que** l'analyseur est conçu pour l'évaluation de valeurs de débit.

6. Dispositif respiratoire selon l'une des revendications 1 à 5, **caractérisé en ce que** l'analyseur est doté d'un comparateur de valeurs de référence.
